# EUROPEAN PATENT APPLICATION

(11) **EP 2 261 231 A1**
(43) Date of publication of application: **15.12.2010**
(21) Application number: 10162698.4
(22) Date of filing: 12.05.2010
(51) Int. Cl.: C07K 1/22, C07K 1/32

(54) **Method and system for protein purification**

(30) Priority: 15.05.2009 US 178816 P
(71) Applicant: Simpson Biotech Co., Ltd., Taoyuan County 333 (TW)
(72) Inventor: Chang, Margaret Dah-Tsyr, 30013, Hsinchu Chinese Taipei (TW); Sheu, Chia-Chin, 333, Kuei Shan Hsiang Chinese Taipei (TW)
(74) Representative: Viering, Jentschura & Partner

(57) **Abstract**

The present invention provides a method and a system for protein purification using a starch binding protein (SBP)-tagged recombinant protein. SBP-binding matrixes are also disclosed in the invention to recover the SBP-tagged recombinant protein. Thus, purifying a target protein is re-usable, convenient and low cost by the present invention.

## Description

### FIELD OF THE INVENTION

The present invention relates to a method and a system for protein purification, and more particularly relates to the method and the system using starch binding protein.

### BACKGROUND OF THE INVENTION

Production of proteins by expression in microbial systems has become a significant source of high value, medically important proteins. Purification and recovery of recombinant proteins are major considerations in the design of a fermentation process. While traditional methods of protein purification can be used to isolate a product, improved methods include the use of recombinant proteins. Recombinant proteins can be purified by affinity column chromatography, the desired component of the recombinant protein being purified by virtue of its covalent attachment to a polypeptide, which binds to an affinity matrix.

Certain systems exist for isolating proteins by the principle of affinity column chromatography.

U.S.Pat. No. 5,643,758 describes a system comprising maltose-binding protein (MBP). A cloned gene is inserted into a pMAL vector down-stream from the malE gene, which encodes MBP. The vector is transformed to a host cell, then the recombinant protein can express in the host cell. The cell lysate or media fraction is loaded to a column containing affinity matrix amylose and washed several times, then using a large amount of maltose to elute the recombinant protein.

U.S. Pat. No. 5,202,247 describes a system comprising cellulose-binding domain. A cellulose column can be used to purify the recombinant protein that contains cellulose-binding domain. The cell lysate or media fraction is loaded to the column and washed. The interaction between cellulose-binding domain and cellulose appears to be driven by hydrophobic interaction at neutral pH. The general method for elution used low polarity solvents such as ethylene glycol, prior to removal of the low polarity solvents by dialysis and filtration.

These current protein purification systems have some disadvantages. The purification processes are inconvenient and laborious. The columns used in purification are expensive. Limitations for protein purification of these systems include unable to isolate the recombinant protein in certain conditions such as EDTA-containing samples as well as the current protein tags being used are relatively large as compared to the target protein bigger than that of this invention.

There are some problems utilizing enzyme products for aquatic feed at present, such as (a) enzyme quickly flows away after feed is added into water, and (b) enzyme activity will be destructed when feed pelleting temperature is higher than 100°C.

### CROSS REFERENCE TO RELATED APPLICATIONS

This application claims priority to the following: U.S. Provisional Patent Application No. 61/178,816 filed on May 15, 2009, U.S. The disclosure of said application is hereby expressly incorporated by reference in its entirety.

### BRIEF DESCRIPTION OF THE DRAWINGS

Figure 1 shows a system designed for highly specific cleavage of fusion proteins followed by the rapid, affinity-based capture and removal of all SBP-tagged residuals (i.e. SBP-tagged endoprotease; SBP-tagged target protein; free SBP).
Figure 2 shows a diagram of SBP-Phytase and Phytase in the water.
Figure 3 shows the result of a releasing experiment of SBP-Phytase in water.
Figure 4 shows the SDS-PAGE result of binding assay of SBD-eGFP to different types of resin. (A) shows the result of amylopectin. (B) shows the result of amylose resin. (C) shows the result of dextrin resin.
Figure 5 shows the SDS-PAGE result of binding assay of SBD-eGFP to alginate beads.
Figure 6 shows the result of affinity-tagged purification and a "Clean-Cut" de-tagging process (LEFT: the result of LZ8; Right: the result of eGFP).
Figure 7 shows the result of purification of immunoglobulin by SBP-SpA amylose resin. (A) shows the result of SBP-SpA purified from *E. coli.* (B) shows the result of SBP-SpA purified from *Pichia.*

### SUMMARY OF THE INVENTION

As embodied and broadly described herein, disclosure herein features starch binding protein (SBP) -tagged recombinant protein, SBP-binding matrix and their uses in protein purification.

In some embodiments, a method is provided for purifying a recombinant protein, comprising
(a) providing a solution including a starch binding protein (SBP)-tagged recombinant protein;
(b) adding the solution into a first container containing a SBP-binding matrix;
(c) eluting an alkaline buffer into the first container of step (b) to obtain a mixture;
(d) mixing the mixture with a solution for cleavage of a SBP and a recombinant protein in a second container to produce a reaction mixture; and
(e) adding the reaction mixture into a third container containing SBP-binding matrix to recover the recombinant protein.

Also, in other embodiments, a system is provided for purifying a recombinant protein comprising
(a) a means for providing a solution including a SBP-tagged recombinant protein;
(b) a means for adding the solution into a first container containing a SBP-binding matrix;
(c) a means for eluting an alkaline buffer into the first container of (b) to obtain a mixture;
(d) a means for mixing the mixture with a solution for cleavage of a SBP and a recombinant protein in a second container to produce a reaction mixture; and
(e) a means for adding the reaction mixture into a third container containing SBP-binding matrix to recover the recombinant protein.

In some aspects, the SBP-binding matrix includes starch, amylopectin, amylose resin, dextrin resin or alginate beads.

In some aspects, the starch binding protein (SBP)-tagged recombinant protein of (a) is from cell and the SBP-binding matrix of (b) is starch.

In other aspects, the first container of (b), the second container of (d) or the third container of (e) is in a disposable form.

In other aspects, the mixture of (d) is further added by SBP-endoprotease. In the specific aspect, the SBP-endoprotease is SBP-SARS protease or SBP-enterokinase.

In another aspect, the solution of (a) has a pH of 4 to 6.

In another aspect, the buffer of (c) has a pH of 7 to 11.

In yet another aspect, the starch binding protein (SBP)-tagged recombinant protein comprises an endoprotease recognition site between SBP and target protein.

In yet another aspect, the solution of (b) has a pH of 4 to 8.

In still another aspect, some embodiments further comprise a neutralizing step before the step (e) for adjusting a pH until 5 to 7.

In still another aspect, some embodiments further comprise a neutralizing means for adjusting a pH until 5 to 7.In some embodiments, a recombinant protein with thermal stability is provided which is prepared by the method of the any of the above-described embodiments, wherein the recombinant protein is fused by a SBP tag and a target protein.

In some aspects, the target protein is Lipase, Xylanase or Phytase.

In other embodiments, a method is provided for preparing a recombinant protein comprising a SBP tag and a protein A, wherein the recombinant protein is expressed by yeast or bacteria.

In certain aspects, the yeast is *Pichia.*

In certain aspects, the bacteria are *E. coli.*

The details of one or more embodiments of the invention are set forth in the accompanying description below. Other features and advantages of the invention will be apparent from the detail descriptions, and from claims.

It is to be understood that both the foregoing general description and the following detailed description are by examples, and are intended to provide further explanation of the invention as claimed.

### DETAILED DESCRIPITION OF THE INVENTION

In describing and claiming the invention, the following terminology will be used in accordance with the definitions set forth below.

As used herein, the term "recombinant protein" is a protein that is derived from recombinant DNA. The term" recombinant DNA "is a form of DNA that does not exist naturally, which is created by combining DNA sequences that would not normally occur together.

The term "starch binding protein" as used herein will be abbreviated as "SBP" and is meant to define all polypeptide having affinity for binding to starch.

The term "SBP tag(s)" used herein is affinity tags of SBP and the term "affinity tags" is tags appended to proteins so that they can be purified from their crude biological source using an affinity technique.

The term "SBP-tagged recombinant protein" as used herein refers to a recombinant protein which has peptide sequences of SBP genetically grafted onto as affinity tags.

The term "SBP-binding matrix" herein refers to any types of matrix that is capable of binding with SBP. Examples of SBP-binding matrix include, but are not limited to, starch, amylopectin, amylose resin, dextrin resin and alginate beads.

As used herein, the term "SBP-endoprotease" refers to any types of endoprotease that is capable of binding with SBP as tags. Endoprotease is an enzyme that cleaves the peptide bonds of nonterminal amino acids (i.e. within the molecule). Examples of SBP-endoprotease include, but are not limited to, SBP-SARS protease or SBP-enterokinase. The practices of this invention are hereinafter described in detail with respect to a method and system for purifying a target protein by use of a re-usable, convenient and low cost SBP-tagged recombinant protein and SBP-binding matrix.

In some embodiments, a method is provided for purifying a recombinant protein, comprising
(a) providing a solution including a starch binding protein (SBP)-tagged recombinant protein;
(b) adding the solution into a first container containing a SBP-binding matrix;
(c) eluting an alkaline buffer into the first container of step (b) to obtain a mixture;
(d) mixing the mixture with a solution for cleavage of a SBP and a recombinant protein in a second container to produce a reaction mixture; and
(e) adding the reaction mixture into a third container containing SBP-binding matrix to recover the recombinant protein.

In some embodiments of the present invention, wherein the SBP-binding matrix includes starch, amylopectin, amylose resin, dextrin resin or alginate beads.

In some embodiments, the present invention provides a method for purifying a recombinant protein comprising the steps of
(a) providing a solution including a starch binding protein (SBP)-tagged recombinant protein from a cell;
(b) adding the solution into a first container containing starch matrix;
(c) eluting an alkaline buffer into the first container of step (b) to obtain a mixture;
(d) mixing the mixture with a solution for cleavage of a SBP and a recombinant protein in a second container to produce a reaction mixture; and
(e) adding the reaction mixture into a third container containing starch matrix to recover the recombinant protein.

In the preferred embodiment, the first container in step (b), the second container in step (d) or the third container in step (e) is in a disposable form. The mixture in step (d) is further added by SBP-endoprotease. In the most preferred embodiment, the SBP-endoprotease is SBP-SARS protease or SBP-enterokinase.

In the preferred embodiment, the solution in step (a) has a pH of 4 to 6, and the buffer in step (c) has a pH of 7 to 11. In the most preferred embodiment, the solution in step (a) has a pH of 5 to 6, and the buffer in step (c) has a pH of 8 to 9. Although the buffer in step (c) has the best discharge effect when pH is 11, excessive alkaline condition may destroy the target proteins. Therefore, recommend pH range of the buffer is from 8 to 9. The buffer can also discharge the protein when pH is of 2 to 4, but the alkaline condition is recommended.

In the preferred embodiment, the starch binding protein (SBP)-tagged recombinant protein has endoprotease recognition site between SBP and target protein. In some embodiment, the solution in step (b) has a pH of 4 to 8 to recover the SBP-tagged recombinant protein. In some preferred embodiment, the pH is 5 to 7 is recommended.

In some embodiments, the method further comprises a neutralizing step before the step (e) for adjusting a pH until 5 to 7. Additionally, the reaction mixture of the third container recover the tag-free recombinant protein while the SBP tag, SBP-endoprotease and undigested SBP-tagged recombinant were captured on SBP-binding matrix.

The present invention also provides a system for purifying a recombinant protein comprising
(a) a means for providing a solution including a SBP-tagged recombinant protein from a cell;
(b) a means for adding the solution into a first container containing SBP-binding matrix;
(c) a means for eluting an alkaline buffer into the first container of (b) to obtain a mixture;
(d) a means for mixing the mixture with a solution for cleavage of a SBP and a recombinant protein in a second container to produce a reaction mixture; and
(e) a means for adding the reaction mixture into a third container containing SBP-binding matrix to recover the recombinant protein.

In some embodiments of the present invention, wherein the SBP-binding matrix includes starch, amylopectin, amylose resin, dextrin resin or alginate beads.

In the preferred embodiment, the first container of (b), the second container of (d) or the third container of (e) is in a disposable form. The mixture of (d) is further added by SBP-endoprotease. In the most preferred embodiment, the SBP-endoprotease is SBP-SARS protease or SBP-enterokinase.

In the preferred embodiment, the solution of (a) has a pH of 4 to 6, and the buffer of (c) has a pH of 7 to 11. In the most preferred embodiment, the solution of (a) has a pH of 5 to 6, and the buffer of (c) has a pH of 8 to 9.

In the preferred embodiment, the starch binding protein (SBP)-tagged recombinant protein has endoprotease recognition site between SBP and target protein. In some embodiments, the solution of (b) has a pH of 4 to 8 to recover the SBP-tagged recombinant protein. In some preferred embodiments, the pH is 5 to 7 is recommended.

In some embodiments, the system further comprises a neutralizing means before the means of (e) for adjusting a pH until 5 to 7. Additionally, the reaction mixture of the third container recover the tag-free recombinant protein while the SBP tag, SBP-endoprotease and undigested SBP-tagged recombinant were captured on SBP-binding matrix.

In some embodiments, a recombinant protein with thermal stability is provided which is prepared by the method of the any of the above-described embodiments, wherein the recombinant protein is fused by a SBP tag and a target protein. In particular embodiments, the target protein is Lipase, Xylanase or Phytase. Thus, the thermal stability of proteins can increase easily and the expand application fields very extensively.

In other embodiments, a method is provided for preparing a recombinant protein comprising a SBP tag and a protein A, wherein the recombinant protein is expressed by yeast or bacteria. In particular embodiments, the yeast is *Pichia pastoris.* In particular embodiments, the bacteria are *E.coli.* The disclosure therefore is useful in any application where purification of a protein is desirable, such as diagnostics, research uses and industrial applications.

### EXAMPLE:

The following example was offered by the way of illustration and not by the way of limitation.

### Example 1 Protein purification

### Construction and expression of SBP-tagged protein

The SBP (starch binding protein) gene was PCR amplified and fused to the N-terminal of target protein with an endoprotease cleavage site between the SBP and target protein gene. The fusion protein gene was then cloned into *Pichia pastoris* expression vector pPICZαA under control of AOX1 promoter and transformed into *Pichia pastoris* GS115 for expression. The *Pichia pastoris* transformant harboring SBP-target protein gene was cultivated in BMGY media for 24 hours. The cells were recovered by centrifugation and resuspended in BMMY containing 0.5% methanol. Methanol (0.5% v/v) was added every 24 hour in order to induce the expression of SBP-tagged recombinant protein. After induction for 5 days, the cells were removed by centrifugation and the cell-free fermentation broth was collected for downstream purification.

### Purification of SBP-tagged recombinant protein by 1^{st} starch column

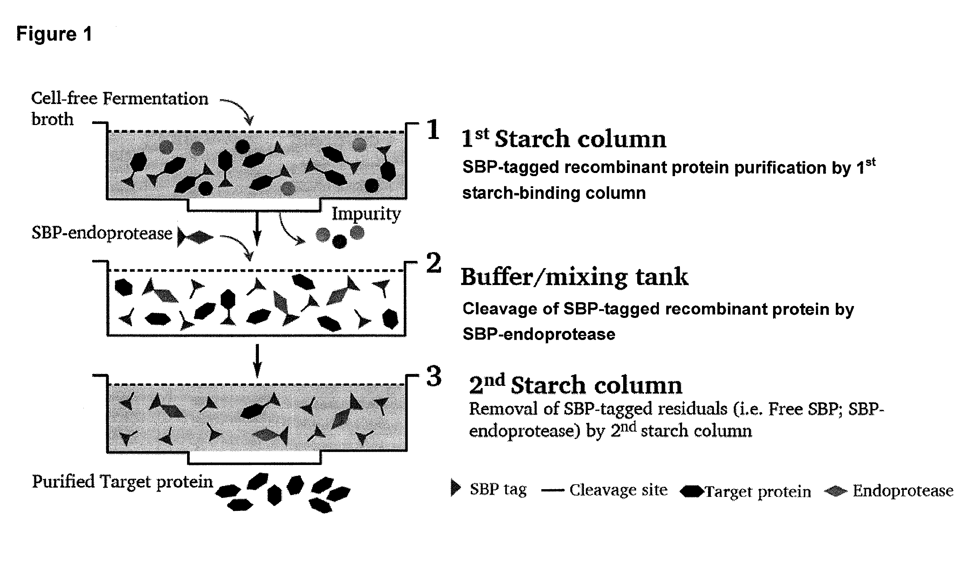

The cell-free fermentation broth was applied to the 1^{st} starch column (Fig. 1). The SBP-tagged recombinant protein was bound on starch and the impurity flew through. The bounded SBP-tagged recombinant protein can be eluted by glycine buffer pH 11 followed by the dialysis against Tris buffer pH 7.4 for storage.

### Endoprotease cleavage of SBP-tagged recombinant protein

The eluted SBP-tagged recombinant protein was mixed with SBP-endoprotease in appropriate buffer. The protease recognition site between SBP-tag and target protein may be cleaved by SBP-protease for SBP-tag removal.

### Purification of tag-free target protein

After SBP-endoprotease treatment, the reaction mixture was applied to the 2^{nd} starch column. The impurities comprising free SBP, SBP-endoprotease and uncut SBP-tagged recombinant protein may be captured by starch column. The completely digested target protein with native N-termini was recovered in the flow through fraction.

### Example 2 Comparison of thermostability

The SBP gene was PCR amplified and fused to the N-termini of target enzyme. The Lipase gene from *R. oryzae,* Xylanase gene from the unpurified ruminal fungal culture and the Phytase gene from *E. coli* were cloned and fused to the SBP. The SBP-Lipase, SBP-Xylanase and SBP-Phytase fusion proteins were expressed by the method described above and used in the present invention.

### Thermal stability of Lipase from different sources

One unit of Lipase activity (U) was defined when the sample released 1µmole p-Nitrophenol per minute in 0.3 mM 4-Nitrophenyl palmitate under 30°C, pH 7.0. SBP-Lipase (52500 U/g) and F-AP15 Lipase (32430 U/g, *R. oryzae* Lipase purchased from Amano Enzyme Inc.) were mixed with fermented soybean meal of water content of 15% or 20% to reach 100U per gram. The mixed 100U/g Lipase from different sources was weighted to pick 12g into 100 ml serum vial. The serum vial with sample therein was locked and autoclaved with 85°C or 90°C for ten minutes. Each condition of each Lipase was tested double times. After treatment, the activity of Lipase was measured. The results were shown in Table 1 and Table 2.

**Table 1 Fermented soybean meal with 15% total water content**

| Sample name | Treating temp. (°C) | Treating time( min) | Percentage (%) |
|---|---|---|---|
| SBP-Lipase | 25°C | 0 | 100% |
| SBP-Lipase | 85°C | 10 | 86.78% |
| SBP-Lipase | 90°C | 10 | 86.2% |
| F-AP 15 | 25°C | 0 | 100% |
| F-AP15 | 85°C | 10 | 66.82% |
| F-AP 15 | 90°C | 10 | 37.02% |

**Table 2 Fermented soybean meal with 20% total water content**

| Sample name | Treating temp.(°C) | Treating time( min) | Percentage(%) |
|---|---|---|---|
| SBP-Lipase | 25°C | 0 | 100% |
| SBP-Lipase | 85°C | 10 | 75% |
| SBP-Lipase | 90°C | 10 | 58% |
| F-AP15 | 25°C | 0 | 100% |
| F-AP15 | 85°C | 10 | 19.61% |
| F-AP15 | 90°C | 10 | 8.56% |

The data showed that the thermal stability of SBP-Lipase was obviously better than F-AP15 Lipase. Under the condition of fermented soybean meal with 15% water content, the residual activity of SBP-Lipase was 86.78% and the residual activity of F-AP15 Lipase was 66.82% after heating 85°C for 10 minutes. After heating 90°C for 10 minutes, the residual activity of SBP-Lipase was still 86.2% but the residual activity of F-AP15 Lipase was just 37.02%.

Under the condition of fermented soybean meal with 20% water content, the residual activity of SBP-Lipase was 75% and the residual activity of F-AP15 Lipase was 19.61% after heating 85°C for 10 minutes. After heating 90°C for 10 minutes, the residual activity of SBP-Lipase was still 58% but the residual activity of F-AP15 Lipase was just 8.56%.

### Thermal stability of Xylanase from different sources

One unit of Xylanase activity (U) was defined when the sample released 1µmole xylose per minute in 1.2 %( w/v) xylan under 50°C, pH 5.3. SBP-Xylanase (3028 U/g) and Xylanase (304 U/g, Xylanase without SBP expressed in *P. pastoris*) were mixed with fermented soybean meal of water content of 15% or 20% to reach 100 U/g. The mixed 100 U/g Xylanase from different sources was weighted to pick 12g into 100 ml serum vial. The serum vial with sample therein was locked and autoclaved with 85°C or 90°C for ten minutes. After treatment, the activity of Xylanase was measured. The results were shown in Table 3 and Table 4.

**Table 3 Fermented soybean meal with 15% total water content**

| Sample name | Treating temp.(°C) | Treating time( min) | Percentage(%) |
|---|---|---|---|
| SBP-Xylanase | 25°C | 0 | 100% |
| SBP-Xylanase | 85°C | 10 | 96.40% |
| SBP-Xylanase | 90°C | 10 | 91.01% |
| Xylanase | 25°C | 0 | 100% |
| Xylanase | 85°C | 10 | 85.43% |
| Xylanase | 90°C | 10 | 79.52% |

**Table 4 Fermented soybean meal with 20% total water content**

| Sample name | Treating temp.(°C) | Treating time( min) | Percentage(%) |
|---|---|---|---|
| SBP-Xylanase | 25°C | 0 | 100% |
| SBP-Xylanase | 85°C | 10 | 93.79% |
| SBP-Xylanase | 90°C | 10 | 86.27% |
| Xylanase | 25°C | 0 | 100% |
| Xylanase | 85°C | 10 | 84% |
| Xylanase | 90°C | 10 | 79% |

The data showed that the thermal stability of SBP-Xylanase was better than Xylanase without SBP-tag. Under the condition of fermented soybean meal with 15% water content, the residual activity of SBP-Xylanase was 96.40% and the residual activity of Xylanase was 85.43% after heating 85°C for 10 minutes. After heating 90°C for 10 minutes, the residual activity of SBP-Xylanase was 91.01% and the residual activity of Xylanase was 79.52%.

Under the condition of fermented soybean meal with 20% water content, the residual activity of SBP-Xylanase was 93.79% and the residual activity of Xylanase was 84% after heating 85°C for 10 minutes. After heating 90°C for 10 minutes, the residual activity of SBP-Xylanase was 86.27% but the residual activity of Xylanase was 79%.

### Thermal stability of Phytase from different sources

One unit of Phytase activity (U) is defined as the amount of enzyme needed for the release of 1µmol inorganic phosphorus (P) from 1.5 mM-sodium phytate per minute at pH 5.0 and 37°C. SBP-Phytase (10500 U/g) and Phytase without SBP (6972 U/g) were mixed with fermented soybean meal of water content of 15% or 20% to reach 100U per gram. The mixed 100U/g Phytase from different sources was weighted to pick 12g into 100 ml serum vial. The serum vial with sample therein was locked and autoclaved with 85°C or 90°C for ten minutes. Each condition of each Phytase was tested double times. After treatment, the activity of Phytase was measured. The results were shown in Table 5 and Table 6.

**Table 5 Fermented soybean meal with 15% total water content**

| Sample name | Treating temp.(°C) | Treating time( min) | Percentage(%) |
|---|---|---|---|
| SBP-Phytase | 25°C | 0 | 100% |
| SBP-Phytase | 85°C | 10 | 88.1% |
| SBP-Phytase | 90°C | 10 | 74.3% |
| Phytase | 25°C | 0 | 100% |
| Phytase | 85°C | 10 | 82.8% |
| Phytase | 90°C | 10 | 59.5% |

**Table 6 Fermented soybean meal with 20% total water content**

| Sample name | Treating temp.(°C) | Treating time( min) | Percentage(%) |
|---|---|---|---|
| SBP-Phytase | 25°C | 0 | 100% |
| SBP-Phytase | 85°C | 10 | 76.6% |
| SBP-Phytase | 90°C | 10 | 48.5% |
| Phytase | 25°C | 0 | 100% |
| Phytase | 85°C | 10 | 55% |
| Phytase | 90°C | 10 | 28.9% |

### Development of aquatic Phytase product

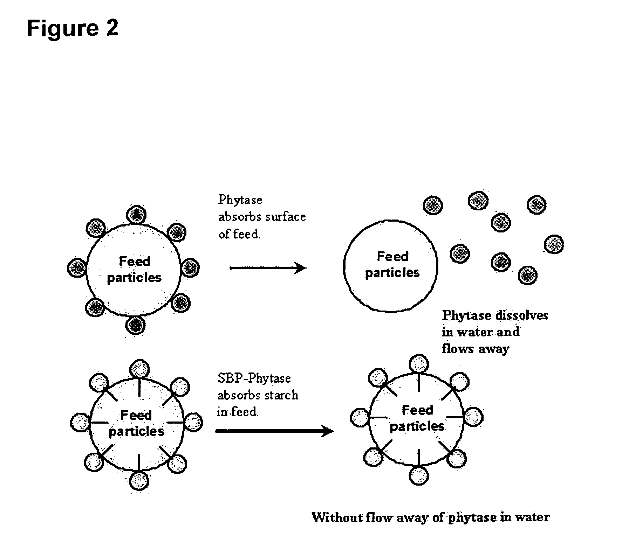

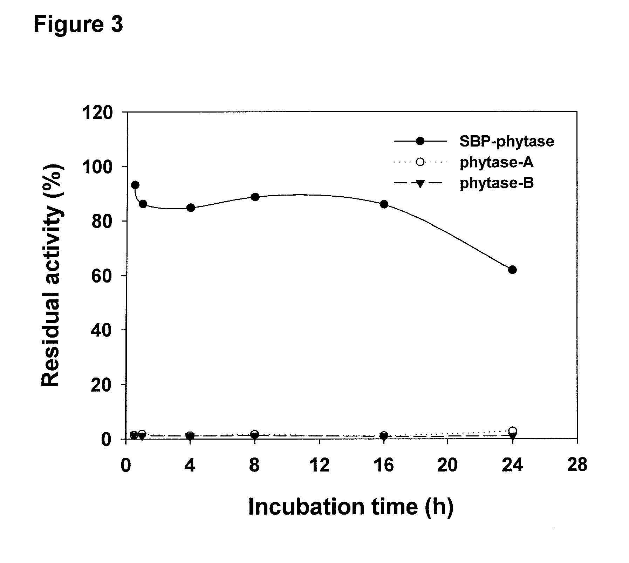

The present invention demonstrated that SBP-Phytase was adapted to being utilized for aquatic feed by (a) absorbing starch to immobilize enzyme on aquatic feed so that the enzyme would not flow away easily when the feed was added into water, and (b) increasing thermostability substantially of the enzyme. Figure 2 shows a diagram of SBP-Phytase and Phytase in the water. Figure 3 shows the result of a releasing experiment of SBP-Phytase in water. 0.4g Phytase powder from different sources was added into 40ml pure water, and vortexed at 25°C. The solution was then centrifuged at 3000 rpm for 30 minutes. 5 ml acetate buffer (pH 6.0) was added to the precipitate and vibrated by an ultrasonic vibrator for 30 minutes. Finally, the activity of Phytase was determined. Phytase of A or B company flowed away in water quickly after they were added into the water. Contrarily, SBP-Phytase still maintained at least 80% activity after being added into water in 16 hours.

### Example 3 Binding assay of SBD-eGFP to different types of SBP matrixes

### Binding assay of SBD-eGFP to different types of resin

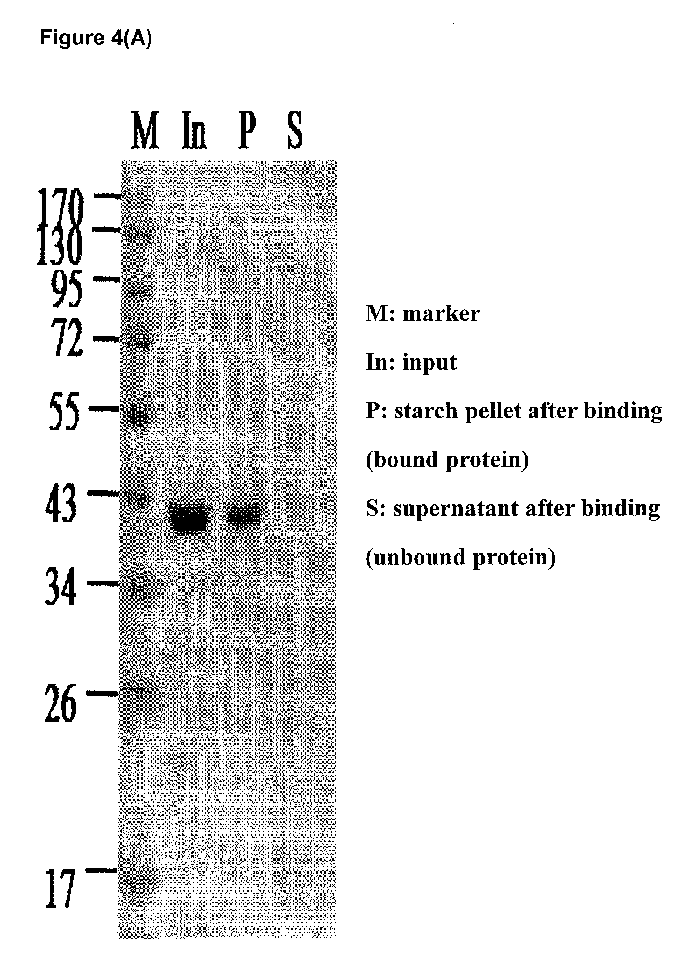

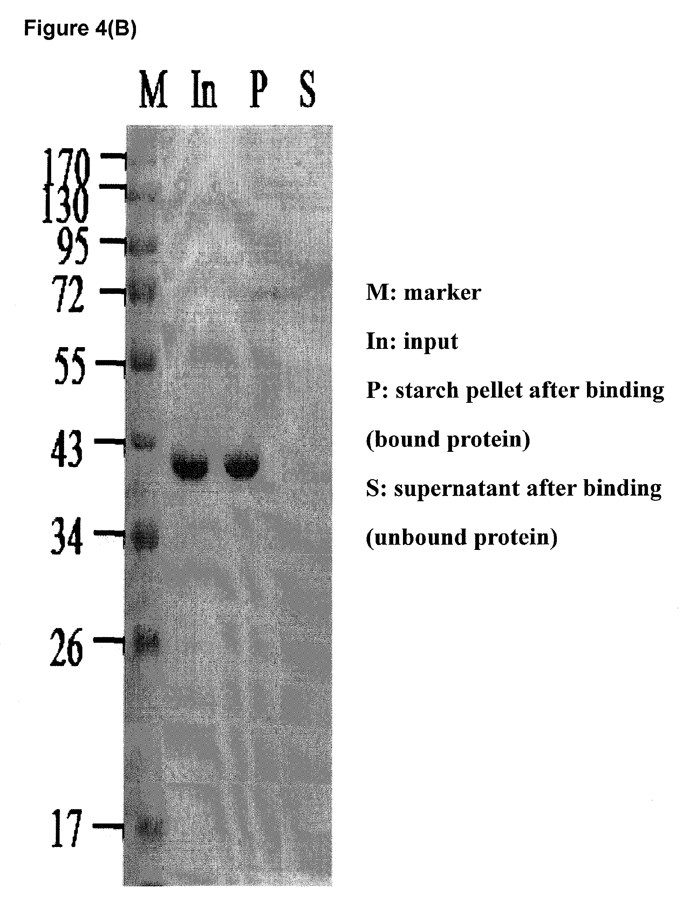

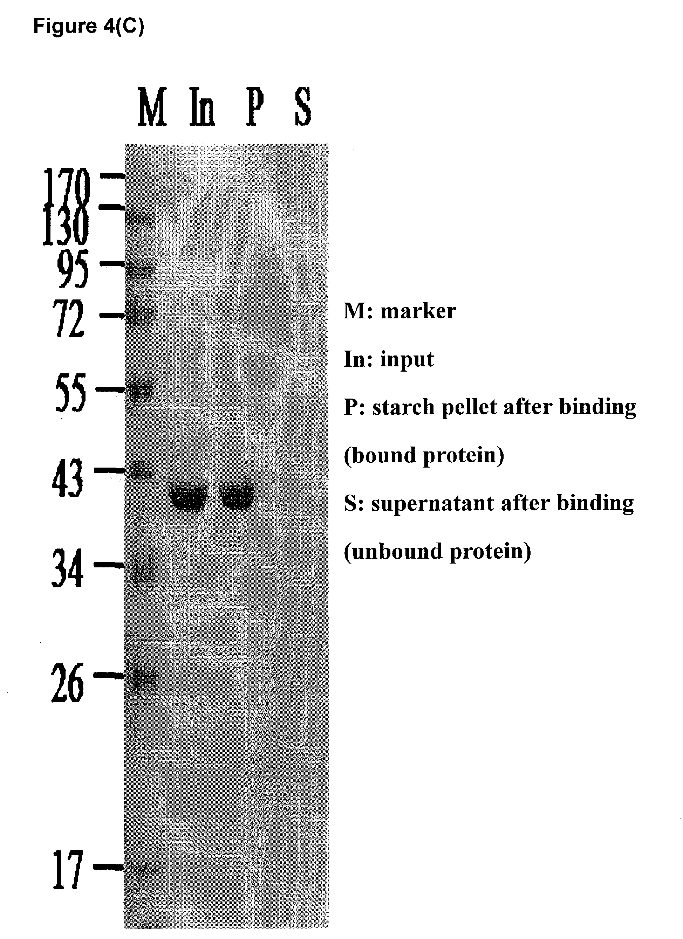

Two milligrams of prewashed amylopectin, amylose resin (Bio-Rad Laboratories Inc., Hercules, California, U.S.), dextrin resin (GE Healthcare., Waukesha, US) and sephadex (Sigma., Saint Louis, Missouri, U.S) were stirred with SBD-eGFP in binding buffer (50 mM NaOAc, pH 5.5) at a concentration of 0.3 mg/mL in a total volume of 200 µL. After incubation with stirring at 25 ° C for 3 hr, the samples were centrifuged. The supernatant (unbound protein) and the resin pellets (bound protein) were then boiled and applied for SDS-PAGE. Results of the binding assay were showed in Figure 4 (A) to (C).

### Binding assay of SBD-eGFP to alginate beads

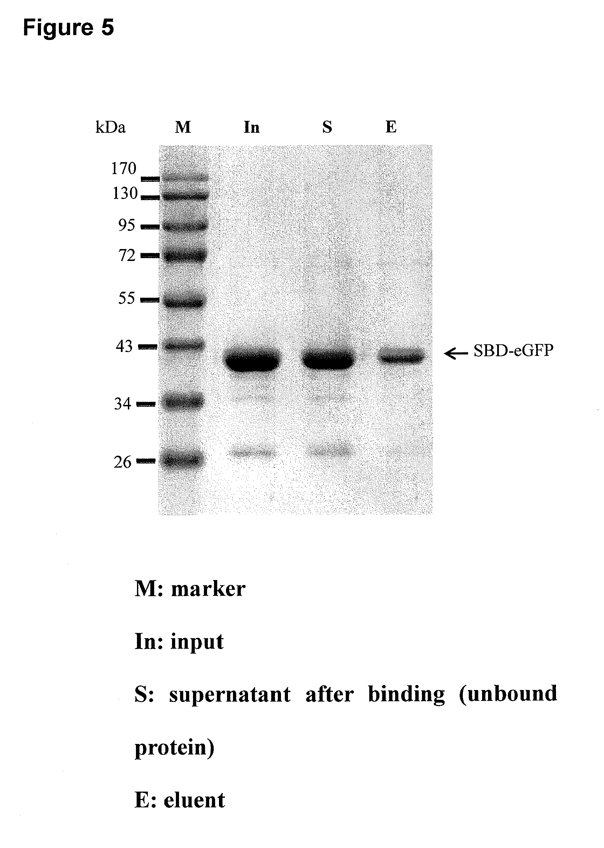

Two hundred and fifty micro-liters of prewashed alginate beads were stirred with SBD-eGFP in binding buffer (50 mM NaOAc, pH 5.5) at a concentration of 0.6 mg/mL in a total volume of 1 mL. After incubation with stirring at 25 °C for 3 hr, the sample was centrifuged. The supernatant (unbound protein) was boiled and applied for SDS-PAGE. The alginate beads were then washed with 1 mL binding buffer. After that, 1 mL elution buffer (10 mM glycine/NaOH, pH 11) was added and stirred with the alginate beads at 25 °C for 30 min. The eluent and the remaining beads were also boiled and applied for SDS-PAGE. Results of the binding assay were showed in Figure 5.

### Example 4 Affinity-tagged purification and a "Clean-Cut" de-tagging process to purify LZ8 (an immune regulator from medical fungus) or eGFP

The SBP gene was PCR amplified and fused to the C-termini of LZ8 (LZ8-eks-SBP) or N-termini of eGFP (SBP-eks-eGFP) with an enterokinase cleavage site (eks) between the SBP and target protein. The SBP gene was PCR amplified and fused to the C-termini of enterokinase (EK-SBP). The LZ8-eks-SBP and EK-SBP were expressed in *P. pastoris* and SBP-eks-eGFP was expressed in insect cell.

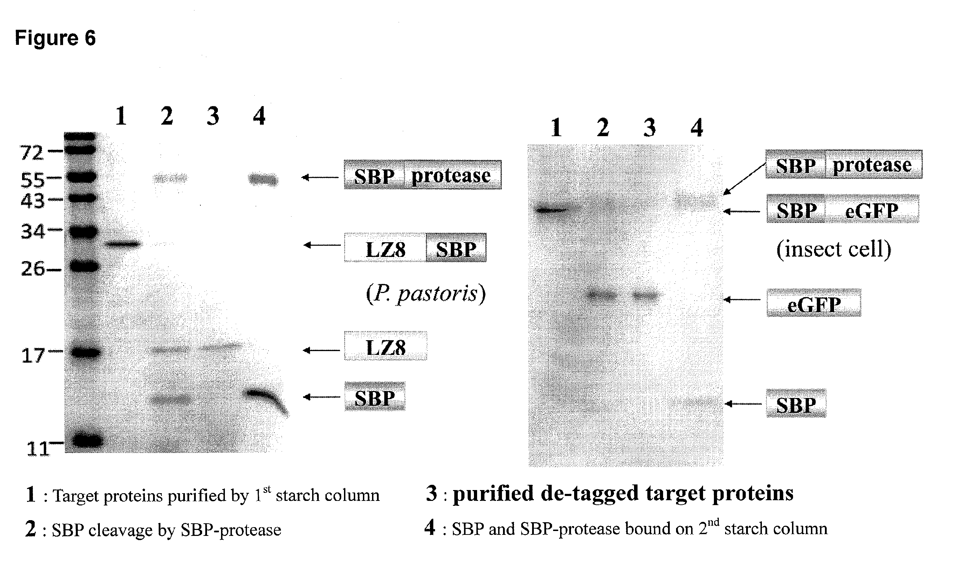

SBP-target proteins (LZ8-eks-SBP or SBP-eks-eGFP) were purified by 1^{st} starch-binding column, and then added SBP-protease (EK-SBP) to cleavage SBP-target proteins in 50 mM sodium acetate (pH5.5) at 37°C for 3 hrs. Applied the mixture to 2^{nd} starch-binding column, flow-through were de-tagged target proteins (LZ8-eks or eGFP), after washing column with 50 mM sodium acetate (pH5.5), SBP-tagged residuals ( Free SBP; EK -SBP and LZ8-eks-SBP , SBP-eks-eGFP) were eluted by 10 mM Glycine/NaOH (pH 11) buffer. The eluent was applied for SDS-PAGE and results were showed in Figure 6 (LEFT: the result of LZ8; Right: the result of eGFP).

### Example 5 Purification of immunoglobulin by SBP-SpA amylose resin

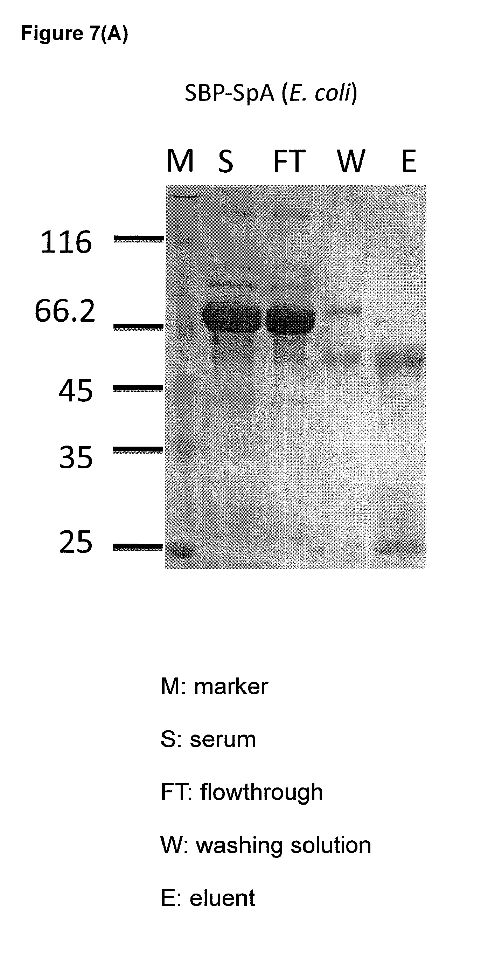

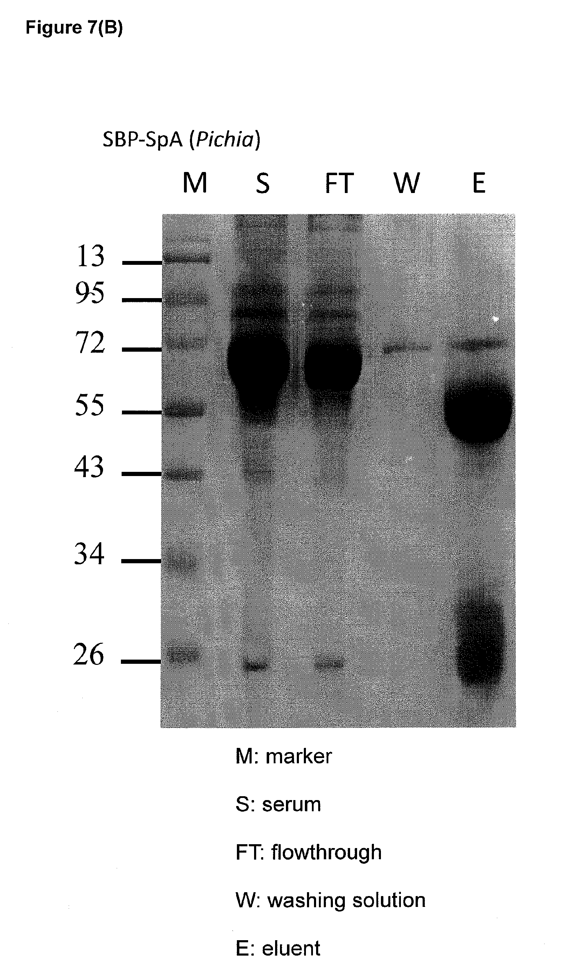

In order to purify IgG, SBP-SpA, a fusion protein of SBP and protein A, purified from *E*. *coli* or *Pichia* was immobilized on CNBr-activated agarose resin (Santa Cruz Biotechnology). Purification of IgG can be carried out by applying the sample to columns of packed SBP-SpA resin. Before the clarified serum applied to the resin, the serum sample should dilute 4 times in binding buffer (100 mM Tris+ 150 mM NaCl, pH 8.5). Applied the sample at a flow rate of 0.5-1ml/min and then washed with 10 column volumes of binding buffer (100 mM Tris+ 150 mM NaCl, pH 8.5). Elution was carried out with 50 mM glycine/HCl, pH 2.5. As soon as the IgG had eluted, neutralized with 1 M Tris, pH 8.0 and condensed 20 times. The eluent was applied for SDS-PAGE and results were showed in Figure 7, wherein (A) showed the result of SBP-SpA purified from *E. coli* and (B) showed the result of SBP-SpA purified from *Pichia.*

The advantages may be summed up as following :
**1. Extremely low production cost:** For example, starch as affinity tag for Phytase (feed enzyme) in no de-tagged process cost less than 1.5 dollars per gram.
**2. Clean-cut (tag-free) proteins:** Clean-cut (tag-free) proteins might be produced by SBP-endoprotease de-tag system disclosed here.
**3. High recovery rate:** The recovery rate of disclosed system was pretty high and the rate was greater than 70%, optionally, the rate was greater than 90% in lab scale.
**4. Very High fermentation titers:** The fermentation titer of disclosed system was pretty high and the rate could be was greater than 10g/l (i.e. *Pichia* expression system).
**5. Successful expressed proteins in many hosts:** Many proteins and enzymes were expressed in many hosts (e.g. *P*. *pastoris, S. cerevisiae, E. coli,* insect cells, yeast or the like).
**6. Poor-expressed proteins can now be obtained:** Poor-expressed proteins can now be obtained by using larger batch plus low-cost starch resin while still under budget.
**7. Disposable protein purified and de-tag sub-system:** There was a disposable protein purify and de-tag sub-system provided in the invention reducing possible contamination.

### OTHER EMBODIMENTS

All of the features disclosed in this specification may be combined in any combination. Each feature disclosed in this specification may be replaced by an alternative feature serving the same, equivalent, or similar purpose. Thus, unless expressly stated otherwise, each feature disclosed is only an example of a generic series of equivalent or similar features. From the above description, one skilled in the art can easily ascertain the essential characteristics of the present invention, and without departing from the spirit and scope thereof, can make various changes and modifications of the invention to adapt it to various usages and conditions. Thus, other embodiments are also within the scope of the following claims.

All patents and publications mentioned in the specification are indicative of the levels of those of ordinary skill in the art to which the invention pertains. All patents and publications are herein incorporated by reference to the same extent as if each individual publication was specifically and individually indicated to be incorporated by reference.

## Claims

1. A method for purifying a recombinant protein comprising
(a) providing a solution including a starch binding protein (SBP)-tagged recombinant protein;
(b) adding the solution into a first container containing a SBP-binding matrix;
(c) eluting an alkaline buffer into the first container of step (b) to obtain a mixture;
(d) mixing the mixture with a solution for cleavage of a SBP and a recombinant protein in a second container to produce a reaction mixture; and
(e) adding the reaction mixture into a third container containing SBP-binding matrix to recover the recombinant protein.

2. The method of claim 1, wherein the SBP-binding matrix includes starch, amylopectin, amylose resin, dextrin resin or alginate beads.

3. The method of claim 1, wherein the starch binding protein (SBP)-tagged recombinant protein in step (a) is from cell and the SBP-binding matrix in step (b) is starch.

4. The method of claim 1, wherein the first container in step (b), the second container in step (d) or the third container in step (e) is in a disposable form.

5. The method of claim 1, wherein the mixture in step (d) is further added by SBP-endoprotease.

6. The method of claim 5, wherein the SBP-endoprotease is SBP-SARS protease or SBP-enterokinase.

7. The method of claim 1, wherein the solution in step (a) has a pH of 4 to 6.

8. The method of claim 1, wherein the buffer in step (c) has a pH of 7 to 11.

9. The method of claim 1, wherein the starch binding protein (SBP)-tagged recombinant protein comprises an endoprotease recognition site between SBP and target protein.

10. The method of claim 1, wherein the solution in step (b) has a pH of 4 to 8.

11. The method of claim 1, further comprising a neutralizing step before the step (e) for adjusting a pH until 5 to 7.

12. A system for purifying a recombinant protein comprising
(a) a means for providing a solution including a SBP-tagged recombinant protein;
(b) a means for adding the solution into a first container containing a SBP-binding matrix;
(c) a means for eluting an alkaline buffer into the first container of (b) to obtain a mixture;
(d) a means for mixing the mixture with a solution for cleavage of a SBP and a recombinant protein in a second container to produce a reaction mixture; and
(e) a means for adding the reaction mixture into a third container containing SBP-binding matrix to recover the recombinant protein.

13. The system of claim 12, wherein the SBP-binding matrix includes starch, amylopectin, amylose resin, or dextrin resin.

14. The system of claim 12, wherein the starch binding protein (SBP)-tagged recombinant protein in the means (a) is from cell and the SBP-binding matrix in the means (b) is starch.

15. The system of claim 12, wherein the first container of (b), the second container in (d) or the third container of (e) is in a disposable form.

16. The system of claim 12, wherein the mixture of (d) is further added by SBP-endoprotease.

17. The system of claim 16, wherein the SBP-endoprotease is SBP-SARS protease or SBP-enterokinase.

18. The system of claim 12, wherein the solution of (a) has a pH of 4 to 6.

19. The system of claim 12, wherein the buffer of (c) has a pH of 7 to 11.

20. The system of claim 12, wherein the starch binding protein (SBP)-tagged recombinant protein comprises an endoprotease recognition site between SBP and target protein.

21. The system of claim 12, wherein the solution of (b) has a pH of 4 to 8.

22. The system of claim 12, further comprising a neutralizing means for adjusting a pH until 5 to 7.

23. A recombinant protein with thermal stability prepared by the method of claim 1, wherein the recombinant protein is fused by a SBP tag and a target protein.

24. The recombinant protein of claim 23, wherein the target protein is Lipase, Xylanase or Phytase.

25. A method for preparing a recombinant protein comprising a SBP tag and a protein A, wherein the recombinant protein is expressed by yeast or bacteria.

26. The method of claim 25, wherein the yeast is *Pichia.*

27. The method of claim 25, wherein the bacteria is *E*. *coli.*
